Europäisches Patentamt

European Patent Office

Office européen des brevets

(19)

(11) Veröffentlichungsnummer: **0 073 987**
**B1**

(12) **EUROPÄISCHE PATENTSCHRIFT**

(45) Veröffentlichungstag der Patentschrift:
16.07.86

(51) Int. Cl.⁴: **C 07 C 25/10**, C 07 C 17/00

(21) Anmeldenummer: **82107618.9**

(22) Anmeldetag: **20.08.82**

(54) **Verfahren zur Herstellung von 1,3,5-Trichlorbenzol.**

(30) Priorität: **02.09.81 DE 3134726**

(43) Veröffentlichungstag der Anmeldung:
**16.03.83 Patentblatt 83/11**

(45) Bekanntmachung des Hinweises auf die Patenterteilung:
**16.07.86 Patentblatt 86/29**

(84) Benannte Vertragsstaaten:
**CH DE FR GB LI**

(56) Entgegenhaltungen:
**DE - C - 947 304**
**DE - C - 2 257 343**

**CHEMISTRY LETTERS, Nr. 1, 1978, Tokio, Y. ONO et al.,
"Low temperature conversion of pentane with aluminum
chloride-metal sulfate mixtures", Seiten 625-628**

(73) Patentinhaber: **BAYER AG, Konzernverwaltung RP
Patentabteilung, D-5090 Leverkusen 1 Bayerwerk (DE)**

(72) Erfinder: **Petruck, Gerd-Michael, Dr.,
Doerpfeldstrasse 49, D-4006 Erkrath (DE)**
Erfinder: **Wambach, Paul Reimund, Dr.,
Berta-von-Suttner-Strasse 65, D-5090 Leverkusen 1 (DE)**

## Beschreibung

Die Erfindung betrifft ein Verfahren zur Herstellung von 1,3,5-Trichlorbenzol durch Isomerisierung von anderen Trichlorbenzolen.

Es ist bekannt, 1,3,5-Trichlorbenzol durch Isomerisierung anderer Trichlorbenzole in Gegenwart von beispielsweise Aluminiumchlorid und Wasser (US-PS 2 866 829), in Gegenwart von Halogeniden des Aluminiums und Wasser oder Alkoholen (DE-PS 947 304), in Gegenwart von Aluminiumchlorid und Magnesiumsulfat (Zh. Org. Khim. 1968, 1247) und in Gegenwart von Aluminiumchlorid und Magnesiumchlorid (CA 63, 9770a) herzustellen.

Nachteilig bei diesen Verfahren ist, dass zur Erzielung der dort angegebenen, jedoch für die technische Anwendung nicht immer befriedigenden Ausbeuten an 1,3,5-Trichlorbenzol grössere Mengen an Katalysator eingesetzt werden. Im übrigen hat sich bei eigenen Nacharbeitungen im Falle der US-PS 2 866 829 gezeigt, dass die dort angegebenen Ausbeuten an 1,3,5-Trichlorbenzol nicht erreicht werden (vgl. den hierzu durchgeführten Vergleichsversuch). Ausserdem werden nur unbefriedigende Ausbeuten an 1,3,5-Trichlorbenzol erzielt, wenn man von reinem 1,2,4-Trichlorbenzol ausgeht. So werden beispielsweise gemäss Beispiel 6 der DE-PS 947 304 nur 14% an 1,3,5-Trichlorbenzol ausgehend von reinem 1,2,4-Trichlorbenzol, erhalten.

Es wurde nun ein Verfahren zur Herstellung von 1,3,5-Trichlorbenzol durch Isomerisierung anderer Trichlorbenzole oder deren Gemische bei erhöhter Temperatur gefunden, das dadurch gekennzeichnet ist, dass man die Isomerisierung in Gegenwart von 0,05 bis 0,5 Mol Aluminiumchlorid pro Mol eingesetztem Trichlorbenzol und 0,9 bis 1,5 Mol Ameisensäure pro Mol Aluminiumchlorid bei Temperaturen von 150 bis 300°C durchführt.

Als Ausgangsprodukte für das erfindungsgemässe Verfahren dienen vor allem 1,2,4- oder 1,2,3-Trichlorbenzol oder Gemische davon. Bevorzugt wird die Isomerisierung mit 1,2,4-Trichlorbenzol durchgeführt.

Das Aluminiumtrichlorid kann in technischer Qualität eingesetzt werden, jedoch ist es häufig vorteilhafter, frisch sublimiertes Aluminiumtrichlorid einzusetzen. Bevorzugt wird das Aluminiumtrichlorid in Mengen von 0,1 bis 0,2 Mol pro Mol eingesetztem Trichlorbenzol verwendet.

Die Ameisensäure, die üblicherweise in Form von reiner Ameisensäure (ca. 95 bis 100gew.-%ig) verwendet wird, wird vorzugsweise in einer Menge von 1,0 bis 1,2 Mol, pro Mol Aluminiumtrichlorid, eingesetzt.

Die Reaktionstemperaturen des erfindungsgemässen Verfahrens liegen bevorzugt bei 200 bis 250°C.

Das erfindungsgemässe Verfahren kann sowohl unter Normaldruck als auch unter Überdruck (bis 50 bar, vorzugsweise 1 bis 20 bar) durchgeführt werden.

Zur Durchführung des erfindungsgemässen Verfahrens kann man zu vorgelegtem Trichlorbenzol die Ameisensäure und das Aluminiumtrichlorid zugeben und das Gemisch im Reaktor unter Feuchtigkeitsausschluss auf die gewünschte Temperatur erwärmen. Zur Aufarbeitung des Reaktionsgemisches wird vom Katalysator abfiltriert, der Rückstand mit Wasser neutral gewaschen und das Filtrat zur Isolierung des reinen 1,3,5-Trichlorbenzols in üblicher Weise destilliert. Die nichtisomerisierten Trichlorbenzole, die z.B. als Destillationsrückstand erhalten werden, können erneut der Isomerisierung zugeführt werden. Durch diese Massnahme kann die Ausbeute an 1,3,5-Trichlorbenzol weiter erhöht werden.

Das erfindungsgemässe Verfahren kann sowohl diskontinuierlich als auch kontinuierlich durchgeführt werden.

Nach dem erfindungsgemässen Verfahren wird das 1,3,5-Trichlorbenzol in besonders guten Ausbeuten erhalten, wobei nur geringe Mengen an Katalysator benötigt werden.

Das 1,3,5-Trichlorbenzol ist ein wertvolles Zwischenprodukt und kann beispielsweise für die Herstellung von Farbstoffen und Pflanzenschutzmitteln verwendet werden (vgl. z.B. DE-OS 1 811 843, DE-OS 2 207 576 und DE-OS 2 149 923).

Beispiele 1 bis 8

Die Beispiele 1 bis 9 sind in der nachfolgenden Tabelle aufgeführt. Aus dieser Tabelle sind auch die Reaktionsbedingungen, die Ausbeuten sowie die prozentuale Zusammensetzung des Rohprodukts zu entnehmen. Die Zusammensetzung des Rohprodukts wurde gaschromatographisch bestimmt.

Die Beispiele 7 bis 9 stellen Vergleichsbeispiele dar, die zeigen, dass der Gehalt an gewünschtem 1,3,5-Trichlorbenzol im Rohprodukt niedriger liegt, wenn die Isomerisierung nicht erfindungsgemäss mit Aluminiumtrichlorid/Ameisensäure durchgeführt wird.

Zur Bestimmung der Ausbeuten an Rohprodukt wurde der Katalysator zusätzlich noch mit Chlorbenzol extrahiert.

| Bei- spiel | Ansatz (g) | Katalysator | Temp. (°C) | Druck (bar) | Zeit (h) | DiCB | Zusammensetzung des Rohproduktes (%) Trichlorbenzol-Isomere | | | | Ausbeute (g) |
|---|---|---|---|---|---|---|---|---|---|---|---|
| | | | | | | | 1, 3, 5– | 1, 2, 4– | 1, 2, 3– | TetraCB | |
| 1 | 363 g 1, 2, 4-Trichlorbenzol | 40,0 g AlCl₃ 15,2 g HCOOH | 250 | 16 | 48 | 5,8 | 29,3 | 52,4 | 5,2 | 7,2 | 290 |
| 2 | 363 g " | 40,0 g AlCl₃ 15,1 g HCOOH | 250 | 16 | 16 | 6,0 | 28,7 | 51,1 | 5,1 | 8,1 | 290 |
| 3 | 363 g " | 53,0 g AlCl 20,2 g HCOOH | 200– 201 | 1 | 24 | 8,9 | 27,4 | 49,0 | 4,8 | 11,2 | 280 |

| Bei-spiel | Ansatz (g) | Katalysator | Temp. (°C) | Druck (bar) | Zeit (h) | DiCB | Zusammensetzung des Rohproduktes (%) Trichlorbenzol-Isomere | | | | Ausbeute (g) |
|---|---|---|---|---|---|---|---|---|---|---|---|
| | | | | | | | 1, 3, 5– | 1, 2, 4– | 1, 2, 3– | TetraCB | |
| 4 | 363 g 1, 2, 4- Trichlorbenzol | 27,0 g AlCl$_3$ 9,7 g HCOOH | 250 | 16 | 24 | 3,9 | 26,3 | 58,9 | 6,0 | 4,9 | 300 |
| 5 | 363 g " | 133,0 g AlCl$_3$ 50,6 g HCOOH | 250 | 16 | 5 | 8,1 | 29,0 | 47,6 | 4,7 | 10,7 | 190 |
| 6 | 363 g " | 26,6 g AlCl$_3$ 9,9 g HCOOH | 250 | 6 | 24 | 5,1 | 27,4 | 55,6 | 5,7 | 6,2 | 301 |
| 7* | 363 g " | 133,0 g AlCl$_3$ 30,0 g MgSO$_4$ | 300 | 16 | 5 | 12,3 | 20,0 | 34,3 | – | 9,3 | 210 |
| 8** | 363 g " | 85,1 g AlCl$_3$ 15,7 g MgCl$_2$ | 180 | 16 | 36 | 4,8 | 23,4 | 59,2 | 5,0 | 7,5 | 220 |
| 9*** | 332 g aus 79% 1, 2, 4- und 21% 1, 2, 3- Isomeren | 60,0 g AlCl$_3$ 8,1 g H$_2$O | 209– 211 | 1 | 24 | 3,6 | 11,5 | 72,8 | 6,8 | 5,3 | 270 |

\* Vergleichsbeispiel: Nach Yu. G. Erykalov et al., Zh. Org. Khim 1968, 1247
\*\* Vergleichsbeispiel: Nach Yu. G. Erykalov et al., CA 63, 9770a
\*\*\* Vergleichsbeispiel: Nach D. Woodruff, US 2 866 829

**Patentansprüche**

1. Verfahren zur Herstellung von 1,3,5-Trichlorbenzol durch Isomerisierung anderer Trichlorbenzole oder deren Gemische bei erhöhter Temperatur, dadurch gekennzeichnet, dass man die Isomerisierung in Gegenwart von 0,05 bis 0,5 Mol Aluminiumtrichlorid pro Mol eingesetztem Trichlorbenzol und 0,9 bis 1,5 Mol Ameisensäure pro Mol Aluminiumtrichlorid bei Temperaturen von 150 bis 300 °C durchführt.

2. Verfahren nach Anspruch 1, dadurch gekennzeichnet, dass man die Isomerisierung in Gegenwart von 0,1 bis 0,2 Mol Aluminiumtrichlorid pro Mol eingesetztem Trichlorbenzol durchführt.

3. Verfahren nach Ansprüchen 1 und 2, dadurch gekennzeichnet, dass man die Isomerisierung in Gegenwart von 1,0 bis 1,2 Mol Ameisensäure pro Mol Aluminiumtrichlorid durchführt.

4. Verfahren nach Ansprüchen 1 bis 3, dadurch gekennzeichnet, dass man die Isomerisierung in Gegenwart von reiner Ameisensäure durchführt.

5. Verfahren nach Ansprüchen 1 bis 4, dadurch gekennzeichnet, dass man die Isomerisierung bei Temperaturen von 200 bis 250 °C durchführt.

**Claims**

1. Process for the preparation of 1,3,5-Trichlorobenzene by the isomerisation of other trichlorobenzenes, or mixtures thereof, at an elevated temperature, characterised in that the isomerisation is carried out in the presence of from 0.05 to 0.5 mol of aluminium trichloride per mol of trichlorobenzene employed, and from 0.9 to 1.5 mols of formic acid per mol of aluminium trichloride, at temperatures of from 150 to 300 °C.

2. Process according to Claim 1, characterised in that the isomerisation is carried out in the presence of from 0.1 to 0.2 mol of aluminium trichloride per mol of trichlorobenzene employed.

3. Process according to Claims 1 and 2, characterised in that the isomerisation is carried out in the presence of from 1.0 to 1.2 mols of formic acid per mol of aluminium trichloride.

4. Process according to Claims 1 to 3, characterised in that the isomerisation is carried out in the presence of pure formic acid.

5. Process according to Claims 1 to 4, characterised in that the isomerisation is carried out at temperatures of from 200 to 250 °C.

**Revendications**

1. Procédé pour la fabrication de 1,3,5-trichlorobenzène par isomérisation d'autres trichlorobenzènes ou de leurs mélanges à température élevée, caractérisé en ce que l'on met en œuvre l'isomérisation en présence de 0,05 à 0,5 mole de trichlorure d'aluminium par mole de trichlorobenzène utilisé et 0,9 à 1,5 mole d'acide formique par mole de trichlorure d'aluminium à des températures de 150 à 300 °C.

2. Procédé selon la revendication 1, caractérisé en ce que l'on met en œuvre l'isomérisation en présence de 0,1 à 0,2 mole de trichlorure d'aluminium par mole de trichlorobenzène utilisé.

3. Procédé selon les revendications 1 et 2, caractérisé en ce que l'on met en œuvre l'isomérisation en présence de 1,0 à 1,2 mole d'acide formique par mole de trichlorure d'aluminium.

4. Procédé selon les revendications 1 à 3, caractérisé en ce que l'on met en œuvre l'isomérisation en présence d'acide formique pur.

5. Procédé selon les revendications 1 à 4, caractérisé en ce que l'on met en œuvre l'isomérisation à des températures de 200 à 250 °C.